# EUROPEAN PATENT APPLICATION

(11) **EP 4 707 291 A1**
(43) Date of publication of application: **11.03.2026**
(21) Application number: 25197954.8
(22) Date of filing: 25.08.2025
(51) Int. Cl.: C07K 5/083, C07K 14/605

(54) **O-TERT-BUTYL-L-SERYL-O-TERT-BUTYL-L-SERYL-GLYCINE AND PREPARATION METHOD THEREOF**

(30) Priority: 29.08.2024 CN 202411203400
(71) Applicant: Sichuan Shifang Sangao Biochemical Industrial Co., Ltd., 618415 Chengdu Sichuan (CN)
(72) Inventor: FANG, Xiaolong, Chengdu, 618415 (CN); LIANG, Hongguo, Chengdu, 618415 (CN); DENG, Jianlong, Chengdu, 618415 (CN); HE, Kaitai, Chengdu, 618415 (CN); YANG, Jian, Chengdu, 618415 (CN); LIU, Mei, Chengdu, 618415 (CN)
(74) Representative: V.O.

(57) **Abstract**

The present application provides O-tert-butyl-L-seryl-O-tert-butyl-L-seryl-glycine and a preparation method thereof. In the preparation process, N-benzyloxycarbonyl-O-tert-butyl-L-serine and O-tert-butyl-L-serine methyl ester are subjected to a condensation reaction to obtain an intermediate compound represented by formula (I), the compound represented by formula (I) and an alkaline compound are subjected to a saponification reaction to obtain an intermediate compound represented by formula (II), then the intermediate compound represented by formula (II) and glycine benzyl ester p-toluenesulfonate are subjected to a condensation reaction to obtain an intermediate compound represented by formula (III), and finally, the intermediate compound represented by formula (III) and hydrogen are subjected to a hydrogenation reaction to obtain the final product. The preparation method provided by the present application simplifies the process, features simple operation and mild reaction conditions, and reduces the difficulty of industrial production, and it does not involve precious raw materials and is conducive to controlling production costs, and the final product has a high purity and a high yield.

## Description

### FIELD

The present application relates to the technical field of synthesis of pharmaceuticals, in particular to an O-tert-butyl-L-seryl-O-tert-butyl-L-seryl-glycine and preparation method thereof.

### BACKGROUND

Tirzepatide is a novel dual receptor agonist of GIP (gastric inhibitory polypeptide) and GLP-1 (glucagon-like peptide-1), which is used for the targeted treatment of diabetes, non-alcoholic steatohepatitis (NASH) and chronic weight management, with a half-life of 116.7 hours. Tirzepatide was developed by Eli Lilly and Company, and the marketing application for tirzepatide injection in China was accepted by the NMPA on September 7, 2022, for improving blood glucose control in adults with type 2 diabetes. On November 8, 2023, the U.S. FDA approved tirzepatide of Eli Lilly and Company for marketing, making it the first dual-target agonist (GIP/GLP-1) obesity treatment drug among similar marketed products. Therefore, tirzepatide has a good development prospect. O-tert-butyl-L-seryl-O-tert-butyl-L-seryl-glycine is an important intermediate in the synthesis of tirzepatide. Tirzepatide is finally obtained through multiple steps of reactions.

At present, there are no literature or patent reports on the preparation method of O-tert-butyl-L-seryl-O-tert-butyl-L-seryl-glycine. Although the applicant obtained the final compound O-tert-butyl-L-seryl-O-tert-butyl-L-seryl-glycine by adopting multi-step synthesis procedures during the research process, it contained many impurities, was not easy to purify, and had a low molar yield.

In view of this, there is an urgent need to develop an industrial synthetic route with simple process, mild reaction conditions and low cost for the preparation of O-tert-butyl-L-seryl-O-tert-butyl-L-seryl-glycine.

### SUMMARY

The technical problem solved by the present application is to provide a method for preparing O-tert-butyl-L-seryl-O-tert-butyl-L-seryl-glycine. This method simplifies the process and features mild reaction conditions. Furthermore, the O-tert-butyl-L-seryl-O-tert-butyl-L-seryl-glycine prepared by the present application has a high purity and a high yield.

In view of this, the present application provides O-tert-butyl-L-seryl-O-tert-butyl-L-seryl-glycine represented by formula (I)

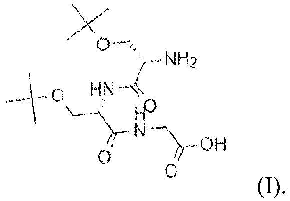

The present application further provides a method for preparing O-tert-butyl-L-seryl-O-tert-butyl-L-seryl-glycine, comprising the following steps:
S1) carrying out a condensation reaction on N-benzyloxycarbonyl-O-tert-butyl-L-serine and O-tert-butyl-L-serine methyl ester under the action of a condensing agent and an organic base to obtain N-benzyloxycarbonyl-O-tert-butyl-L-seryl-O-tert-butyl-L-serine methyl ester represented by formula (II);
S2) carrying out a saponification reaction on the N-benzyloxycarbonyl-O-tert-butyl-L-seryl-O-tert-butyl-L-serine methyl ester and an alkaline compound to obtain N-benzyloxycarbonyl-O-tert-butyl-L-seryl-O-tert-butyl-L-serine represented by formula (III);
S3) carrying out a condensation reaction on the N-benzyloxycarbonyl-O-tert-butyl-L-seryl-O-tert-butyl-L-serine and glycine benzyl ester p-toluenesulfonate under the action of a condensing agent and an organic base to obtain N-benzyloxycarbonyl-O-tert-butyl-L-seryl-O-tert-butyl-L-seryl-glycine benzyl ester represented by formula (IV);
S4) carrying out a hydrogenation reaction on the N-benzyloxycarbonyl-O-tert-butyl-L-seryl-O-tert-butyl-L-seryl-glycine benzyl ester and hydrogen under the action of a catalyst to obtain O-tert-butyl-L-seryl-O-tert-butyl-L-seryl-glycine represented by formula (I);

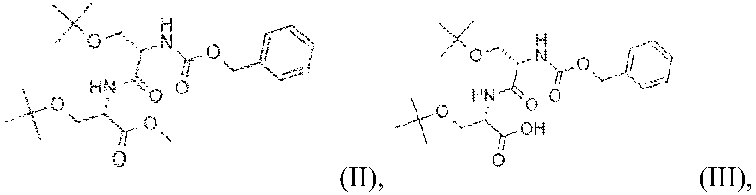

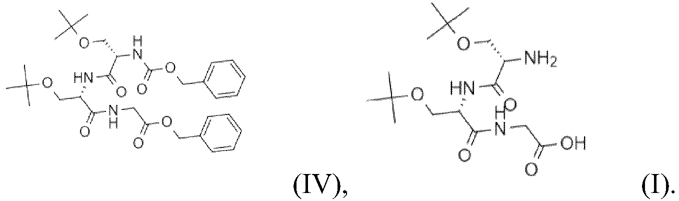

Preferably, step S1) specifically is
mixing the N-benzyloxycarbonyl-O-tert-butyl-L-serine, the O-tert-butyl-L-serine methyl ester and the organic base with an organic solvent, then adding the condensing agent to carry out the condensation reaction to obtain the N-benzyloxycarbonyl-O-tert-butyl-L-seryl-O-tert-butyl-L-serine methyl ester.

Preferably, in step S1), a molar ratio of the N-benzyloxycarbonyl-O-tert-butyl-L-serine, the O-tert-butyl-L-serine methyl ester, the condensing agent, and the organic base is 1: (1-2): (1-1.5): (1-1.5).

Preferably, the condensing agent is selected from the group consisting of EDCl/HOBT, EDCl/HOSU, DCC/HOSU, HATU, HBTU and CDI, and the organic base is selected from the group consisting of triethylamine and N, N-diisopropylethylamine.

Preferably, the condensing agent is added at a temperature of 0-5°C, and the condensation reaction is carried out at a temperature of 0-50°C for 12-24 h.

Preferably, the organic solvent is selected from the group consisting of ethyl acetate, dichloromethane, acetonitrile and tetrahydrofuran.

Preferably, step S2) specifically is:
mixing the N-benzyloxycarbonyl-O-tert-butyl-L-seryl-O-tert-butyl-L-serine methyl ester with an organic solvent, then adding a sodium hydroxide solution to carry out the saponification reaction to obtain the N-benzyloxycarbonyl-O-tert-butyl-L-seryl-O-tert-butyl-L-serine.

Preferably, in step S2), a molar ratio of the N-benzyloxycarbonyl-O-tert-butyl-L-seryl-O-tert-butyl-L-serine methyl ester to the sodium hydroxide is 1: (1-2).

Preferably, in step S2), the sodium hydroxide solution is added at a temperature of 0-5°C, and the saponification reaction is carried out at a temperature of 0-40°C for 1-6 h.

Preferably, in step S2), a concentration of the sodium hydroxide solution is 5-60%.

Preferably, in step S2), the organic solvent is selected from the group consisting of acetonitrile, tetrahydrofuran, methanol and ethanol.

Preferably, step S3) specifically is:
mixing the N-benzyloxycarbonyl-O-tert-butyl-L-seryl-O-tert-butyl-L-serine, the glycine benzyl ester p-toluenesulfonate and the organic base with an organic solvent, then adding the condensing agent to carry out the condensation reaction to obtain the N-benzyloxycarbonyl-O-tert-butyl-L-seryl-O-tert-butyl-L-seryl-glycine benzyl ester.

Preferably, in step S3), a molar ratio of the N-benzyloxycarbonyl-O-tert-butyl-L-seryl-O-tert-butyl-L-serine, the glycine benzyl ester p-toluenesulfonate, the organic base, and the condensing agent is 1: (1-2): (1-1.5): (1-1.5).

Preferably, in step S3), the condensing agent is selected from the group consisting of EDCl/HOBT, EDCl/HOSU, DCC/HOSU, HATU, HBTU and CDI, and the organic base is selected from the group consisting of trimethylamine and N, N-diisopropylethylamine.

Preferably, in step S3), the condensing agent is added at a temperature of 0-5°C, and the condensation reaction is carried out at a temperature of 0-50°C for 12-24 h.

Preferably, in step S3), the organic solvent is selected from the group consisting of ethyl acetate, dichloromethane, acetonitrile and tetrahydrofuran.

Preferably, step S4) specifically is:
mixing the N-benzyloxycarbonyl-O-tert-butyl-L-seryl-O-tert-butyl-L-seryl-glycine benzyl ester with an organic solvent, adding the catalyst, and then introducing hydrogen to carry out the hydrogenation reaction to obtain the O-tert-butyl-L-seryl-O-tert-butyl-L-seryl-glycine.

Preferably, in step S4), a mass ratio of the N-benzyloxycarbonyl-O-tert-butyl-L-seryl-O-tert-butyl-L-seryl-glycine benzyl ester to the catalyst is 1: (0.01-0.2).

Preferably, in step S4), the hydrogenation reaction is carried out at a temperature of 20-30°C for 20-24 h.

Preferably, the organic solvent is selected from the group consisting of acetonitrile, tetrahydrofuran/water, methanol, ethanol, acetone/water and ethyl acetate/water, and the catalyst is selected from the group consisting of Pd/C, Raney nickel and Pd/BaSO₄.

The present application provides a method for preparing O-tert-butyl-L-seryl-O-tert-butyl-L-seryl-glycine, comprising first carrying out a condensation reaction on N-benzyloxycarbonyl-O-tert-butyl-L-serine and O-tert-butyl-L-serine methyl ester to obtain N-benzyloxycarbonyl-O-tert-butyl-L-seryl-O-tert-butyl-L-serine methyl ester, then carrying out a saponification reaction on the N-benzyloxycarbonyl-O-tert-butyl-L-seryl-O-tert-butyl-L-serine methyl ester with an alkaline compound, carrying out a condensation reaction on the resulting product with glycine benzyl ester p-toluenesulfonate, and finally carrying out a hydrogenation reaction on the resulting product with hydrogen to obtain O-tert-butyl-L-seryl-O-tert-butyl-L-seryl-glycine. In the present application, in the above process of preparing O-tert-butyl-L-seryl-O-tert-butyl-L-seryl-glycine, N-benzyloxycarbonyl-O-tert-butyl-L-serine is used as a raw material, and the tirzepatide intermediate O-tert-butyl-L-seryl-O-tert-butyl-L-seryl-glycine is synthesized through four steps, namely, sequential condensation reaction, saponification reaction, condensation reaction, and hydrogenation reaction, which simplifies the process, features simple operation and mild reaction conditions. Furthermore, the present application improves the yield and purity of O-tert-butyl-L-seryl-O-tert-butyl-L-seryl-glycine by regulating the specific process conditions in the above preparation steps.

### BRIEF DESCRIPTION OF DRAWINGS

FIG. 1 is an HPLC chromatogram of O-tert-butyl-L-seryl-O-tert-butyl-L-seryl-glycine prepared in Example 2 of the present application.
Figure 2 is a hydrogen nuclear magnetic resonance spectrum of O-tert-butyl-L-seryl-O-tert-butyl-L-seryl-glycine prepared in Example 2 of the present application in CD₃OD.
Figure 3 is a hydrogen nuclear magnetic resonance spectrum of O-tert-butyl-L-seryl-O-tert-butyl-L-seryl-glycine prepared in Example 2 of the present application in D₂O.
Figure 4 is a hydrogen nuclear magnetic resonance spectrum of O-tert-butyl-L-seryl-O-tert-butyl-L-seryl-glycine prepared in Example 2 of the present application in DMSO.

### DETAILED DESCRIPTION

To further understand the present application, the following describes the preferred embodiments of the present application in conjunction with examples. However, it should be understood that these descriptions are only for further illustrating the features and advantages of the present application, and not for limiting the claims of the present application.

Considering the demand in the prior art for an industrial synthetic route with a simple preparation process, mild reaction conditions, and low cost for the synthesis of tirzepatide intermediates, the present application provides O-tert-butyl-L-seryl-O-tert-butyl-L-seryl-glycine and preparation method thereof. In the preparation process, N-benzyloxycarbonyl-O-tert-butyl-L-serine, as a raw material, is reacted with other reagents through four steps of a condensation reaction, a saponification reaction, a condensation reaction, and a hydrogenation reaction to synthesize the tirzepatide intermediate O-tert-butyl-L-seryl-O-tert-butyl-L-seryl-glycine. This preparation method simplifies the process route, features simple operation and mild reaction conditions, and reduces the difficulty of industrial production. In the preparation process, by controlling the process conditions during preparation, the yield and purity of the tirzepatide intermediate O-tert-butyl-L-seryl-O-tert-butyl-L-seryl-glycine are further improved.

First, the present application provides O-tert-butyl-L-seryl-O-tert-butyl-L-seryl-glycine represented by formula (I)

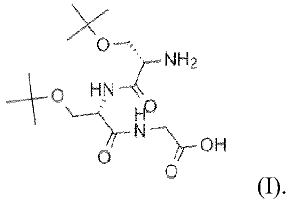

Further, in an embodiment, the present application provides a method for preparing O-tert-butyl-L-seryl-O-tert-butyl-L-seryl-glycine, comprising the following steps:
S1) carrying out a condensation reaction on N-benzyloxycarbonyl-O-tert-butyl-L-serine and O-tert-butyl-L-serine methyl ester under the action of a condensing agent and an organic base to obtain N-benzyloxycarbonyl-O-tert-butyl-L-seryl-O-tert-butyl-L-serine methyl ester represented by formula (II);
S2) carrying out a saponification reaction on the N-benzyloxycarbonyl-O-tert-butyl-L-seryl-O-tert-butyl-L-serine methyl ester and an alkaline compound to obtain N-benzyloxycarbonyl-O-tert-butyl-L-seryl-O-tert-butyl-L-serine represented by formula (III);
S3) carrying out a condensation reaction on the N-benzyloxycarbonyl-O-tert-butyl-L-seryl-O-tert-butyl-L-serine and glycine benzyl ester p-toluenesulfonate under the action of a condensing agent and an organic base to obtain N-benzyloxycarbonyl-O-tert-butyl-L-seryl-O-tert-butyl-L-seryl-glycine benzyl ester represented by formula (IV);
S4) carrying out a hydrogenation reaction on the N-benzyloxycarbonyl-O-tert-butyl-L-seryl-O-tert-butyl-L-seryl-glycine benzyl ester and hydrogen under the action of a catalyst to obtain O-tert-butyl-L-seryl-O-tert-butyl-L-seryl-glycine represented by formula (I);

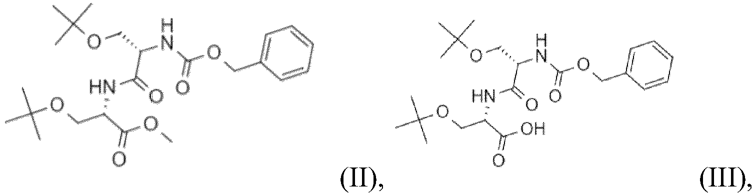

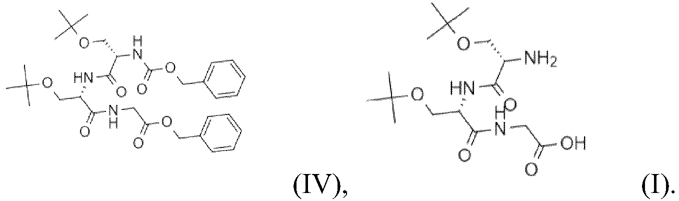

In the present application, in the preparation process of the tirzepatide intermediate O-tert-butyl-L-seryl-O-tert-butyl-L-seryl-glycine, N-benzyloxycarbonyl-O-tert-butyl-L-serine and O-tert-butyl-L-serine methyl ester are used as raw materials to carry out the condensation reaction under the conditions of the condensing agent and the organic base, thereby obtaining the N-benzyloxycarbonyl-O-tert-butyl-L-seryl-O-tert-butyl-L-serine methyl ester.

The process specifically comprises the following steps: mixing the N-benzyloxycarbonyl-O-tert-butyl-L-serine, the O-tert-butyl-L-serine methyl ester and the organic base with an organic solvent uniformly, then adding the condensing agent and stirring to carry out the condensation reaction, and carrying out post-treatment to obtain the intermediate compound N-benzyloxycarbonyl-O-tert-butyl-L-seryl-O-tert-butyl-L-serine methyl ester as an oily substance.

The reaction formula of the above process is specifically as follows:

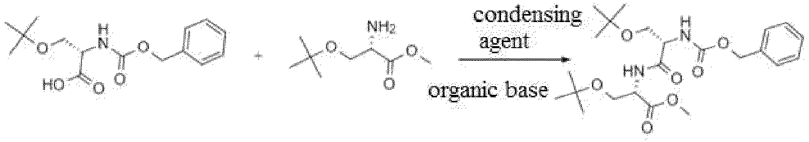

In the above process, a molar ratio of the N-benzyloxycarbonyl-O-tert-butyl-L-serine, the O-tert-butyl-L-serine methyl ester, the condensing agent, and the organic base is 1: (1-2): (1-1.5): (1-1.5). Specifically, the molar ratio of the N-benzyloxycarbonyl-O-tert-butyl-L-serine, the O-tert-butyl-L-serine methyl ester, the condensing agent, and the organic base is 1: (1.2-1.8): (1.1-1.4): (1.1-1.4). More specifically, the molar ratio of the N-benzyloxycarbonyl-O-tert-butyl-L-serine, the O-tert-butyl-L-serine methyl ester, the condensing agent, and the organic base is 1: 1.5: 1.2: 1.4. The condensing agent is added at a temperature of 0-5°C. Specifically, the condensing agent is added at a temperature of 1-3°C. If the addition temperature of the condensing agent is too high, it will lead to an increase in impurities. The condensation reaction is carried out at a temperature of 0-50°C. Specifically, the condensation reaction is carried out at a temperature of 0-10°C, 10-20°C, 20-25°C, 25-30°C, 30-35°C, 35-40°C or 40-50°C. Preferably, the condensation reaction is carried out at a temperature of 25-35°C. If the temperature of the condensation reaction is too low, the reaction is incomplete, resulting in low yield, and if the temperature of the condensation reaction is too high, the purity is reduced. The condensation reaction is carried out for 12-24h. Specifically, the condensation reaction is carried out for 18-20h. The organic base is selected from the group consisting of triethylamine and N, N-diisopropylethylamine. The condensing agent is selected from the group consisting of EDCl/HOBT, EDCl/HOSU, DCC/HOSU, HATU, HBTU and CDI. Further, the condensing agent is selected from the group consisting of EDCl/HOBT, EDCl/HOSU, HATU and HBTU. The condensing agent selected from the above reagents can make the yield and purity of the product higher. In the above condensing agents, EDCl/HOBT means that EDCl and HOBT are added simultaneously, and similarly, EDCl/HOSU also means that EDCl and HOSU are added simultaneously. The organic solvent is selected from the group consisting of ethyl acetate, dichloromethane, acetonitrile and tetrahydrofuran. Specifically, the organic solvent is selected from the group consisting of ethyl acetate and acetonitrile. Under the same conditions, the selection of the above organic solvent is beneficial to improve the yield and purity of the product.

In the present application, the above intermediate product N-benzyloxycarbonyl-O-tert-butyl-L-seryl-O-tert-butyl-L-serine methyl ester and the alkaline compound are subjected to the saponification reaction to obtain the N-benzyloxycarbonyl-O-tert-butyl-L-seryl-O-tert-butyl-L-serine.

The process specifically comprises the following steps: mixing the N-benzyloxycarbonyl-O-tert-butyl-L-seryl-O-tert-butyl-L-serine methyl ester with the organic solvent uniformly, then adding the sodium hydroxide solution and stirring to carry out the saponification reaction, and carrying out post-treatment to obtain the intermediate compound the N-benzyloxycarbonyl-O-tert-butyl-L-seryl-O-tert-butyl-L-serine as an oily substance.

The reaction formula of the above process is specifically as follows:

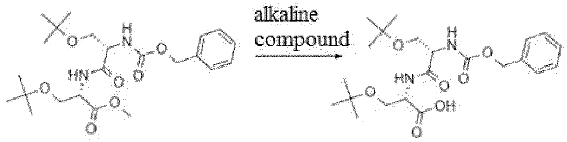

In the above process, a molar ratio of the N-benzyloxycarbonyl-O-tert-butyl-L-seryl-O-tert-butyl-L-serine methyl ester to the sodium hydroxide is 1: (1-2). Specifically, the molar ratio of the N-benzyloxycarbonyl-O-tert-butyl-L-seryl-O-tert-butyl-L-serine methyl ester to the sodium hydroxide is 1: (1.2-1.8). More specifically, the molar ratio of the N-benzyloxycarbonyl-O-tert-butyl-L-seryl-O-tert-butyl-L-serine methyl ester to the sodium hydroxide is 1: 1.5. The sodium hydroxide solution is added at a temperature of 0-5°C. Specifically, the sodium hydroxide solution is added at a temperature of 3-4°C. When the sodium hydroxide solution is added, the system temperature is increased, and if the adding temperature of the sodium hydroxide solution is not controlled, the reaction temperature is overhigh and impurities are increased. A concentration of the sodium hydroxide solution is 5-60%. Specifically, the concentration of the sodium hydroxide solution is 5-30%. More specifically, the concentration of the sodium hydroxide solution is 10%, 20% and 30%. If the concentration of the sodium hydroxide is too high, the purity of the product will be reduced. The saponification reaction is carried out at a temperature of 0-40°C. Specifically, the saponification reaction is carried out at a temperature of 0-10°C, 10-15°C, 15-20°C or 20-25°C. Further, the saponification reaction is carried out at a temperature of 15-25°C. More specifically, the saponification reaction is carried out at a temperature of 15-20°C or 20-25°C. If the temperature of the saponification reaction is low, the reaction time is long, impurities are increased. The above temperature can ensure quick reaction, excessive impurities are not increased, and the purity of the product is ensured. The saponification reaction is carried out for 1-6 h. Specifically, the saponification reaction is carried out for 2-4 h. More specifically, the saponification reaction is carried out for 3h or 4 h. The organic solvent is selected from the group consisting of acetonitrile, tetrahydrofuran, methanol and ethanol.

According to the present application, the obtained product N-benzyloxycarbonyl-O-tert-butyl-L-seryl-O-tert-butyl-L-serine and glycine benzyl ester p-toluenesulfonate are subjected to the condensation reaction under the action of the condensing agent and the organic base to obtain the N-benzyloxycarbonyl-O-tert-butyl-L-seryl-O-tert-butyl-L-seryl-glycine benzyl ester.

The process specifically comprises the following steps: mixing the N-benzyloxycarbonyl-O-tert-butyl-L-seryl-O-tert-butyl-L-serine, the glycine benzyl ester p-toluenesulfonate and the organic base with the organic solvent, then adding the condensing agent and mixing to carry out the condensation reaction, and carrying out post-treatment to obtain the intermediate compound N-benzyloxycarbonyl-O-tert-butyl-L-seryl-O-tert-butyl-L-seryl-glycine benzyl ester as an oily substance.

The reaction formula of the above process is specifically as follows:

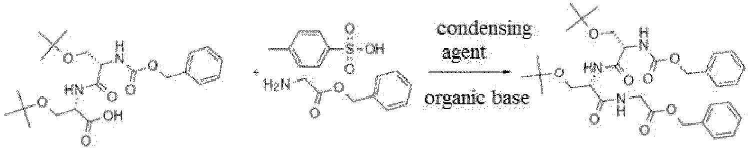

In the above process, a molar ratio of the N-benzyloxycarbonyl-O-tert-butyl-L-seryl-O-tert-butyl-L-serine, the glycine benzyl ester p-toluenesulfonate, the condensing agent and the organic base is 1: (1-2): (1-1.5): (1-1.5). Specifically, the molar ratio of the N-benzyloxycarbonyl-O-tert-butyl-L-seryl-O-tert-butyl-L-serine, the glycine benzyl ester p-toluenesulfonate, the condensing agent and the organic base is 1: (1.2-1.8): (1.1-1.4): (1.1-1.4). More specifically, the molar ratio of the N-benzyloxycarbonyl-O-tert-butyl-L-seryl-O-tert-butyl-L-serine, the glycine benzyl ester p-toluenesulfonate, the condensing agent and the organic base is 1:1.5:1.2:1.4. In the present application, the glycine benzyl ester p-toluenesulfonate is used as a key raw material. If glycine methyl ester hydrochloride or other materials are used as reaction raw materials, the reaction steps will be increased, and the final product will have more impurities, be difficult to purify and have lower molar yield. The condensing agent is added at a temperature controlled to be 0-5°C. Specifically, the condensing agent is added at a temperature controlled to be 2-4°C. If the addition temperature of the condensing agent is too high, it will lead to an increase in impurities. The condensation reaction is carried out at a temperature of 0-50°C. Specifically, the condensation reaction is carried out at a temperature of 0-10°C, 10-20°C, 20-25°C, 25-30°C, 30-35°C, 35-40°C or 40-50°C. Preferably, the condensation reaction is carried out at a temperature of 25-35°C. Further, the condensation reaction is carried out at a temperature of 30-35°C. If the temperature of the condensation reaction is too low, the reaction is not complete, resulting in a low yield, and if the temperature of the condensation reaction is too high, resulting in a low purity. The condensation reaction is carried out for 12-24 h. Specifically, the condensation reaction is carried out for 18-20 h. The organic base is selected from the group consisting of trimethylamine and N, N-diisopropylethylamine. The condensing agent is selected from the group consisting of EDCl/HOBT, EDCl/HOSU, DCC/HOSU, HATU, HBTU and CDI. Further, the condensing agent is selected from the group consisting of EDCl/HOBT, EDCl/HOSU, HATU and HBTU. The condensing agent selected from the above reagents can make the yield and purity of the product higher. In the above condensing agents, EDCl/HOBT means that EDCl and HOBT are added simultaneously, and similarly, EDCl/HOSU also means that EDCl and HOSU are added simultaneously. The organic solvent is selected from the group consisting of ethyl acetate, dichloromethane, acetonitrile and tetrahydrofuran. Specifically, the organic solvent is selected from the group consisting of ethyl acetate and acetonitrile. Under the same conditions, the selection of the above organic solvent is beneficial to improve the yield and purity of the product. In the present application, the condensation reaction in this process and the condensation reaction in the step S1) may be performed under the same or different process conditions, which is not particularly limited in this application.

In the present application, finally, the obtained intermediate product N-benzyloxycarbonyl-O-tert-butyl-L-seryl-O-tert-butyl-L-seryl-glycine benzyl ester and hydrogen are subjected to the hydrogenation reaction under the action of the catalyst to obtain O-tert-butyl-L-seryl-O-tert-butyl-L-seryl-glycine.

The process specifically comprises the following steps: mixing the N-benzyloxycarbonyl-O-tert-butyl-L-seryl-O-tert-butyl-L-seryl-glycine benzyl ester with the organic solvent uniformly, adding the catalyst, introducing hydrogen under stirring to carry out the hydrogenation reaction, and carrying out post-treatment to obtain the target compound O-tert-butyl-L-seryl-O-tert-butyl-L-seryl-glycine. The reaction formula of the above process is specifically as follows:

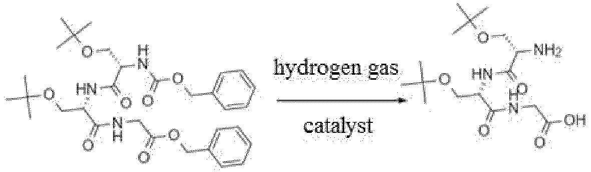

In the above process, a mass ratio of the N-benzyloxycarbonyl-O-tert-butyl-L-seryl-O-tert-butyl-L-seryl-glycine benzyl ester to the catalyst is 1: (0.01-0.2). Specifically, the mass ratio of the N-benzyloxycarbonyl-O-tert-butyl-L-seryl-O-tert-butyl-L-seryl-glycine benzyl ester to the catalyst is 1: (0.07-0.15). More specifically, the mass ratio of the N-benzyloxycarbonyl-O-tert-butyl-L-seryl-O-tert-butyl-L-seryl-glycine benzyl ester to the catalyst is 1: 0.1. The hydrogenation reaction is carried out at a temperature of 20-30°C. Specifically, the hydrogenation reaction is carried out at a temperature of 23-27°C. If the temperature of the hydrogenation reaction is too low, the reaction time is long, and if the reaction temperature is too high, impurities are increased, so that the impurities are not easy to remove. The hydrogenation reaction is carried out for 20-24 h. Specifically, the hydrogenation reaction is carried out for 21-23 h. The organic solvent is selected from the group consisting of acetonitrile, tetrahydrofuran/water, methanol, ethanol, acetone/water and ethyl acetate/water. Specifically, the organic solvent is methanol. The catalyst is selected from the group consisting of Pd/C, Raney nickel and Pd/BaSO₄. Specifically, the catalyst is Pd/C.

For further understanding of the present application, the following examples are given to illustrate the preparation of O-tert-butyl-L-seryl-O-tert-butyl-L-seryl-glycine according to the present application, and the scope of the present application is not limited by the following examples.

The raw materials used in the application are all industrial raw materials which can be purchased in the market.

### Example 1

### (1) Preparation of N-benzyloxycarbonyl-O-tert-butyl-L-seryl-O-tert-butyl-L-serine methyl ester

100g of N-benzyloxycarbonyl-O-tert-butyl-L-serine was added into 800g of ethyl acetate, and stirred to dissolve completely, and then 90g of O-tert-butyl-L-serine methyl ester and 51.3g of trimethylamine were added. After stirring uniformly, the mixture was cooled to 3°C, and then 9.1g of HOBT and 78g of EDCl were added. The mixture was stirred to react for 30 minutes, and heated to 33°C to react for 18 hours. TLC showed that N-carbobenzoxy-O-tert-butyl-L-serine had completely reacted. 400g of water was added to the reaction solution to quench the reaction, and then liquids were separated. Ethyl acetate layer was collected. The ethyl acetate layer was washed with 6N hydrochloric acid aqueous solution, 5% sodium bicarbonate aqueous solution and saturated sodium chloride aqueous solution respectively. After the washing was completed, the ethyl acetate layer was dried over sodium sulfate, filtered, and concentrated to dryness to obtain 168g of N-benzyloxycarbonyl-O-tert-butyl-L-seryl-O-tert-butyl-L-serine methyl ester as an oily substance.

### (2) Preparation of N-benzyloxycarbonyl-O-tert-butyl-L-seryl-O-tert-butyl-L-serine

168g of the N-benzyloxycarbonyl-O-tert-butyl-L-seryl-O-tert-butyl-L-serine methyl ester as the oily substance was added into 150g of tetrahydrofuran and 300g of water. The mixture was cooled to 4°C, and 67.6g of 30% sodium hydroxide aqueous solution was slowly added. After stirring uniformly, the mixture was heated to 23°C to react for 3 hours. TLC showed that N-benzyloxycarbonyl-O-tert-butyl-L-seryl-O-tert-butyl-L-serine methyl ester had completely reacted. 400g of ethyl acetate was added into the system. The pH value of the system was adjusted to 2-3 by using a 6N hydrochloric acid aqueous solution, and then liquids were separated. The ethyl acetate layer was collected. The ethyl acetate layer was washed with saturated sodium chloride for 3 times. After the washing was completed, the ethyl acetate layer was dried over sodium sulfate, filtered, and concentrated to dryness to obtain 152g of N-benzyloxycarbonyl-O-tert-butyl-L-seryl-O-tert-butyl-L-serine as an oily substance.

### (3) Preparation of N-benzyloxycarbonyl-O-tert-butyl-L-seryl-O-tert-butyl-L-seryl-glycine benzyl ester

152g of N-benzyloxycarbonyl-O-tert-butyl-L-seryl-O-tert-butyl-L-serine was added into 1200g of ethyl acetate, and stirred to dissolve completely, and then 121g of glycine benzyl ester p-toluenesulfonate and 48g of triethylamine were added. After stirring uniformly, the mixture was cooled to 2°C, and then 9.3g of HOBT and 79g of EDCl were added. The mixture was stirred to react for 30 minutes, and heated to 33°C to react for 18 hours. TLC showed that N-benzyloxycarbonyl-O-tert-butyl-L-seryl-O-tert-butyl-L-serine had completely reacted. 400g of water was added to the reaction solution to quench the reaction, and then liquids were separated. Ethyl acetate layer was collected. The ethyl acetate layer was washed with 6N hydrochloric acid aqueous solution, 5% sodium bicarbonate aqueous solution and saturated sodium chloride aqueous solution respectively. After the washing was completed, the ethyl acetate layer was dried over sodium sulfate, filtered, and concentrated to dryness to obtain 177g of N-benzyloxycarbonyl-O-tert-butyl-L-seryl-O-tert-butyl-L-seryl-glycine benzyl ester as an oily substance.

### (4) Preparation of O-tert-butyl-L-seryl-O-tert-butyl-L-seryl-glycine

177g of N-benzyloxycarbonyl-O-tert-butyl-L-seryl-O-tert-butyl-L-seryl-glycine benzyl ester was added to 1060g of methanol, and stirred to dissolve completely, and then 17.7g of Pd/C was added. The temperature was controlled at 23°C. The system was replaced with nitrogen, and then hydrogen was introduced. After reacting for 23 hours, TLC showed that N-benzyloxycarbonyl-O-tert-butyl-L-seryl-O-tert-butyl-L-seryl-glycine benzyl ester had completely reacted. After filtering out the Pd/C, the filtrate was concentrated until a large amount of solid precipitated. The system was cooled to 2°C for crystallization for 3 hours. The filter cake was rinsed with a small amount of methanol, and dried by blowing at 50°C to dryness to obtain 89g of O-tert-butyl-L-seryl-O-tert-butyl-L-seryl-glycine. The results show that: molar yield: 73%; HPLC: 99.48%; isomers: not detected.

### Example 2

### Preparation of N-benzyloxycarbonyl-O-tert-butyl-L-seryl-O-tert-butyl-L-serine methyl ester

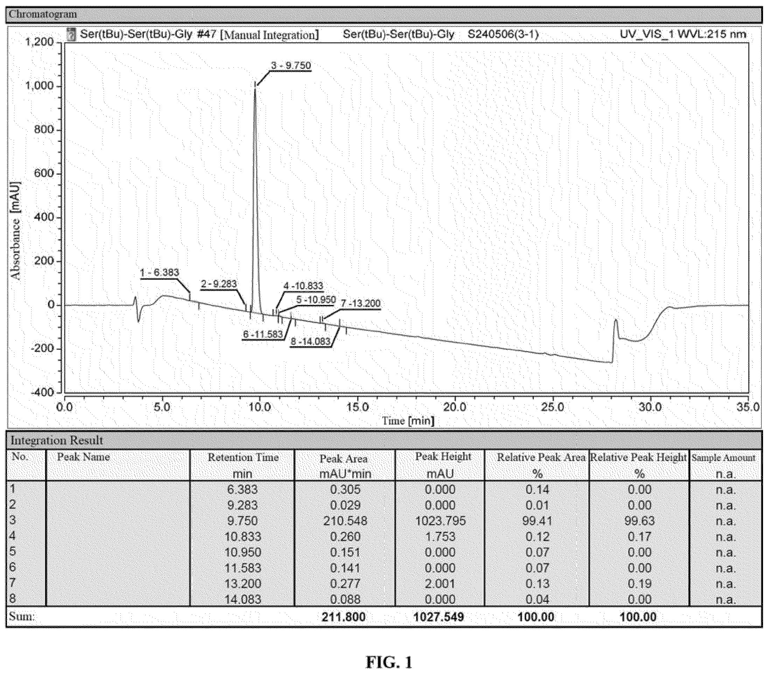

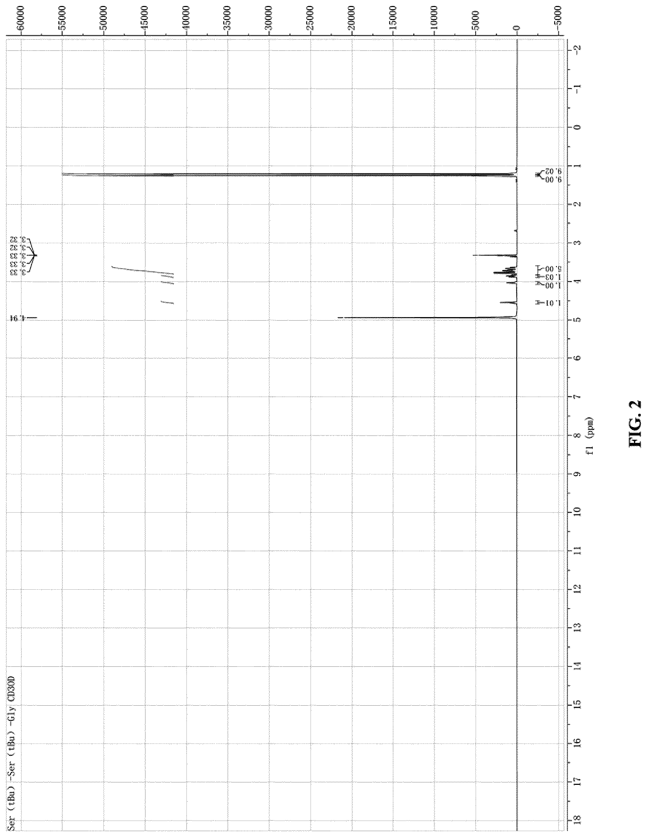

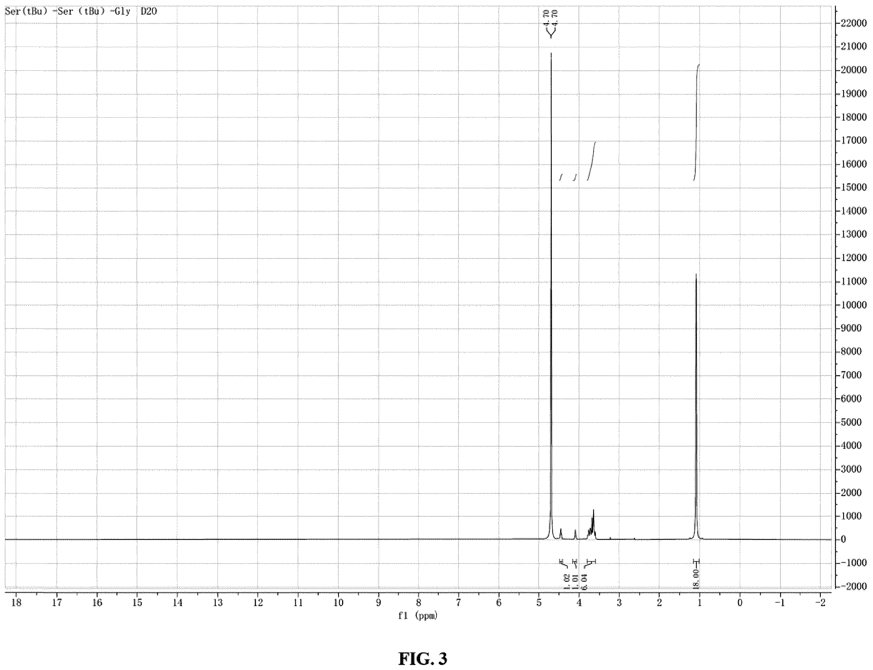

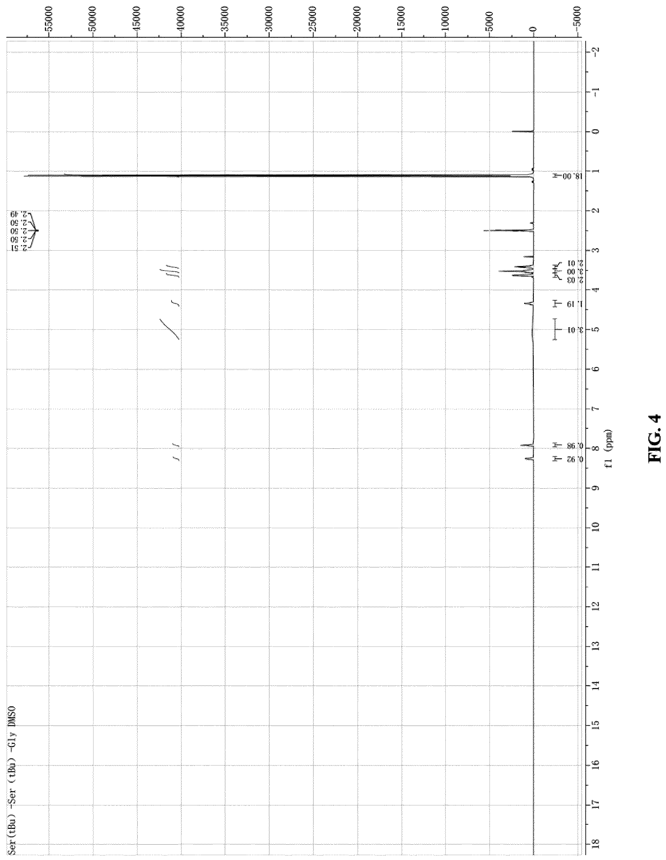

(1) 1.7kg of N-benzyloxycarbonyl-O-tert-butyl-L-serine was added into 13.6kg of ethyl acetate, and stirred to dissolve completely, and then 1.62kg of O-tert-butyl-L-serine methyl ester and 0.87kg of trimethylamine were added. After stirring uniformly, the mixture was cooled to 1°C, and then 0.155kg of HOBT and 1.32kg of EDCl were added. The mixture was stirred to react for 30 minutes, and heated to 33°C to react for 18 hours. TLC showed that N-carbobenzoxy-O-tert-butyl-L-serine had completely reacted. 7kg of water was added to the reaction solution to quench the reaction, and then liquids were separated. Ethyl acetate layer was collected. The ethyl acetate layer was washed with 6N hydrochloric acid aqueous solution, 5% sodium bicarbonate aqueous solution and saturated sodium chloride aqueous solution respectively. After the washing was completed, the ethyl acetate layer was dried over sodium sulfate, filtered, and concentrated to dryness to obtain 2.4kg of N-benzyloxycarbonyl-O-tert-butyl-L-seryl-O-tert-butyl-L-serine methyl ester as an oily substance.
(2) Preparation of N-benzyloxycarbonyl-O-tert-butyl-L-seryl-O-tert-butyl-L-serine
   2.4kg of the N-benzyloxycarbonyl-O-tert-butyl-L-seryl-O-tert-butyl-L-serine methyl ester as the oily substance was added into 4.8kg of tetrahydrofuran and 12kg of water. The mixture was cooled to 3°C, and 1kg of 30% sodium hydroxide aqueous solution was slowly added. After stirring uniformly, the mixture was heated to 22°C to react for 3 hours. TLC showed that N-benzyloxycarbonyl-O-tert-butyl-L-seryl-O-tert-butyl-L-serine methyl ester had completely reacted. 6kg of ethyl acetate was added into the system. The pH value of the system was adjusted to 2-3 by using a 6N hydrochloric acid aqueous solution, and then liquids were separated. The ethyl acetate layer was collected. The ethyl acetate layer was washed with saturated sodium chloride for 3 times. After the washing was completed, the ethyl acetate layer was dried over sodium sulfate, filtered, and concentrated to dryness to obtain 2.2kg of N-benzyloxycarbonyl-O-tert-butyl-L-seryl-O-tert-butyl-L-serine as an oily substance.
(3) Preparation of N-benzyloxycarbonyl-O-tert-butyl-L-seryl-O-tert-butyl-L-seryl-glycine benzyl ester
   2.2kg of N-benzyloxycarbonyl-O-tert-butyl-L-seryl-O-tert-butyl-L-serine was added into 17.4kg of ethyl acetate, and stirred to dissolve completely, and then 1.927kg of glycine benzyl ester p-toluenesulfonate and 0.72kg of triethylamine were added. After stirring uniformly, the mixture was cooled to 4°C, and then 0.134kg of HOBT and 1.043kg of EDCl were added. The mixture was stirred to react for 30 minutes, and heated to 33°C to react for 18 hours. TLC showed that N-benzyloxycarbonyl-O-tert-butyl-L-seryl-O-tert-butyl-L-serine had completely reacted. 10kg of water was added to the reaction solution to quench the reaction, and then liquids were separated. Ethyl acetate layer was collected. The ethyl acetate layer was washed with 6N hydrochloric acid aqueous solution, 5% sodium bicarbonate aqueous solution and saturated sodium chloride aqueous solution respectively. After the washing was completed, the ethyl acetate layer was dried over sodium sulfate, filtered, and concentrated to dryness to obtain 2.8kg of N-benzyloxycarbonyl-O-tert-butyl-L-seryl-O-tert-butyl-L-seryl-glycine benzyl ester as an oily substance.
(4) Preparation of O-tert-butyl-L-seryl-O-tert-butyl-L-seryl-glycine
   2.8kg of N-benzyloxycarbonyl-O-tert-butyl-L-seryl-O-tert-butyl-L-seryl-glycine benzyl ester was added to 12kg of methanol, and stirred to dissolve completely, and then 0.196kg of Pd/C was added. The temperature was controlled at 23°C. The system was replaced with nitrogen, and then hydrogen was introduced. After reacting for 23 hours, TLC showed that N-benzyloxycarbonyl-O-tert-butyl-L-seryl-O-tert-butyl-L-seryl-glycine benzyl ester had completely reacted. After filtering out the Pd/C, the filtrate was concentrated until a large amount of solid precipitated. The system was cooled to 4°C for crystallization for 3 hours. The filter cake was rinsed with a small amount of methanol, and dried by blowing at 50°C to dryness to obtain 1.477kg of O-tert-butyl-L-seryl-O-tert-butyl-L-seryl-glycine. The results show that: molar yield: 71%; HPLC: 99.41% (as shown in Fig. 1); isomers: not detected. The nuclear magnetic resonance spectra are shown in Figs. 2, 3, and 4. Figs. 2, 3, and 4 are the proton spectra of the final product in different solvents, respectively.

### Example 3

### (1) Preparation of N-benzyloxycarbonyl-O-tert-butyl-L-seryl-O-tert-butyl-L-serine methyl ester

100g of N-benzyloxycarbonyl-O-tert-butyl-L-serine was added into 800g of ethyl acetate, and stirred to dissolve completely, and then 90g of O-tert-butyl-L-serine methyl ester and 61.2g of N, N-diisopropylethylamine were added. After stirring uniformly, the mixture was cooled to 3°C, and then 9.1g of HOBT and 78g of EDCI were added. The mixture was stirred to react for 30 minutes, and heated to 33°C to react for 18 hours. TLC showed that N-carbobenzoxy-O-tert-butyl-L-serine had completely reacted. 400g of water was added to the reaction solution to quench the reaction, and then liquids were separated. Ethyl acetate layer was collected. The ethyl acetate layer was washed with 6N hydrochloric acid aqueous solution, 5% sodium bicarbonate aqueous solution and saturated sodium chloride aqueous solution respectively. After the washing was completed, the ethyl acetate layer was dried over sodium sulfate, filtered, and concentrated to dryness to obtain 164g of N-benzyloxycarbonyl-O-tert-butyl-L-seryl-O-tert-butyl-L-serine methyl ester as an oily substance.

### (2) Preparation of N-benzyloxycarbonyl-O-tert-butyl-L-seryl-O-tert-butyl-L-serine

164g of the N-benzyloxycarbonyl-O-tert-butyl-L-seryl-O-tert-butyl-L-serine methyl ester as the oily substance was added into 160g of tetrahydrofuran and 300g of water. The mixture was cooled to 4°C, and 72.4g of 30% sodium hydroxide aqueous solution was slowly added. After stirring uniformly, the mixture was heated to 23°C to react for 3 hours. TLC showed that N-benzyloxycarbonyl-O-tert-butyl-L-seryl-O-tert-butyl-L-serine methyl ester had completely reacted. 400g of ethyl acetate was added into the system. The pH value of the system was adjusted to 2-3 by using a 6N hydrochloric acid aqueous solution, and then liquids were separated. The ethyl acetate layer was collected. The ethyl acetate layer was washed with saturated sodium chloride for 3 times. After the washing was completed, the ethyl acetate layer was dried over sodium sulfate, filtered, and concentrated to dryness to obtain 150g of N-benzyloxycarbonyl-O-tert-butyl-L-seryl-O-tert-butyl-L-serine as an oily substance.

### (3) Preparation of N-benzyloxycarbonyl-O-tert-butyl-L-seryl-O-tert-butyl-L-seryl-glycine benzyl ester

150g of N-benzyloxycarbonyl-O-tert-butyl-L-seryl-O-tert-butyl-L-serine was added into 1200g of ethyl acetate, and stirred to dissolve completely, and then 133g of glycine benzyl ester p-toluenesulfonate and 61.8g of N, N-diisopropylethylamine were added. After stirring uniformly, the mixture was cooled to 2°C, and then 9.3g of HOBT and 79g of EDCl were added. The mixture was stirred to react for 30 minutes, and heated to 33°C to react for 18 hours. TLC showed that N-benzyloxycarbonyl-O-tert-butyl-L-seryl-O-tert-butyl-L-serine had completely reacted. 400g of water was added to the reaction solution to quench the reaction, and then liquids were separated. Ethyl acetate layer was collected. The ethyl acetate layer was washed with 6N hydrochloric acid aqueous solution, 5% sodium bicarbonate aqueous solution and saturated sodium chloride aqueous solution respectively. After the washing was completed, the ethyl acetate layer was dried over sodium sulfate, filtered, and concentrated to dryness to obtain 178g of N-benzyloxycarbonyl-O-tert-butyl-L-seryl-O-tert-butyl-L-seryl-glycine benzyl ester as an oily substance.

### (4) Preparation of O-tert-butyl-L-seryl-O-tert-butyl-L-seryl-glycine

178g of N-benzyloxycarbonyl-O-tert-butyl-L-seryl-O-tert-butyl-L-seryl-glycine benzyl ester was added to 1060g of methanol, and stirred to dissolve completely, and then 17.8g of Pd/C was added. The temperature was controlled at 23°C. The system was replaced with nitrogen, and then hydrogen was introduced. After reacting for 23 hours, TLC showed that N-benzyloxycarbonyl-O-tert-butyl-L-seryl-O-tert-butyl-L-seryl-glycine benzyl ester had completely reacted. After filtering out the Pd/C, the filtrate was concentrated until a large amount of solid precipitated. The system was cooled to 2°C for crystallization for 3 hours. The filter cake was rinsed with a small amount of methanol, and dried by blowing at 50°C to dryness to obtain 86g of O-tert-butyl-L-seryl-O-tert-butyl-L-seryl-glycine. The results show that: molar yield: 70%; HPLC: 99.43%; isomers: not detected.

### Example 4

### (1) Preparation of N-benzyloxycarbonyl-O-tert-butyl-L-seryl-O-tert-butyl-L-serine methyl ester

100g of N-benzyloxycarbonyl-O-tert-butyl-L-serine was added into 800g of ethyl acetate, and stirred to dissolve completely, and then 90g of O-tert-butyl-L-serine methyl ester and 51.3g of trimethylamine were added. After stirring uniformly, the mixture was cooled to 3°C, and then 9.1g of HOBT and 78g of EDCl were added. The mixture was reacted at 0-5°C for 18 hours. TLC showed that N-carbobenzoxy-O-tert-butyl-L-serine had not completely reacted. 400g of water was added to the reaction solution to quench the reaction, and then liquids were separated. Ethyl acetate layer was collected. The ethyl acetate layer was washed with 6N hydrochloric acid aqueous solution, 5% sodium bicarbonate aqueous solution and saturated sodium chloride aqueous solution respectively. After the washing was completed, the ethyl acetate layer was dried over sodium sulfate, filtered, and concentrated to dryness to obtain 79g of N-benzyloxycarbonyl-O-tert-butyl-L-seryl-O-tert-butyl-L-serine methyl ester as an oily substance.

### (2) Preparation of N-benzyloxycarbonyl-O-tert-butyl-L-seryl-O-tert-butyl-L-serine

79g of the N-benzyloxycarbonyl-O-tert-butyl-L-seryl-O-tert-butyl-L-serine methyl ester as the oily substance was added into 80g of tetrahydrofuran and 150g of water. The mixture was cooled to 4°C, and 31g of 30% sodium hydroxide aqueous solution was slowly added. After stirring uniformly, the mixture was reacted at 0-5°C for 23h. TLC showed that N-benzyloxycarbonyl-O-tert-butyl-L-seryl-O-tert-butyl-L-serine methyl ester still had not completely reacted. 400g of ethyl acetate was added into the system. The pH value of the system was adjusted to 2-3 by using a 6N hydrochloric acid aqueous solution, and then liquids were separated. The ethyl acetate layer was collected. The ethyl acetate layer was washed with saturated sodium chloride for 3 times. After the washing was completed, the ethyl acetate layer was dried over sodium sulfate, filtered, and concentrated to dryness to obtain 71g of N-benzyloxycarbonyl-O-tert-butyl-L-seryl-O-tert-butyl- L-serine as an oily substance.

### (3) Preparation of N-benzyloxycarbonyl-O-tert-butyl-L-seryl-O-tert-butyl-L-seryl-glycine benzyl ester

71g of N-benzyloxycarbonyl-O-tert-butyl-L-seryl-O-tert-butyl-L-serine was added into 560g of ethyl acetate, and stirred to dissolve completely, and then 56g of glycine benzyl ester p-toluenesulfonate and 22g of triethylamine were added. After stirring uniformly, the mixture was cooled to 2°C, and then 4.3g of HOBT and 37g of EDCl were added. The mixture was reacted at 0-5°C for 18 hours. TLC showed that N-benzyloxycarbonyl-O-tert-butyl-L-seryl-O-tert-butyl-L-serine had not completely reacted. 200g of water was added to the reaction solution to quench the reaction, and then liquids were separated. Ethyl acetate layer was collected. The ethyl acetate layer was washed with 6N hydrochloric acid aqueous solution, 5% sodium bicarbonate aqueous solution and saturated sodium chloride aqueous solution respectively. After the washing was completed, the ethyl acetate layer was dried over sodium sulfate, filtered, and concentrated to dryness to obtain 41g of N-benzyloxycarbonyl-O-tert-butyl-L-seryl-O-tert-butyl-L-seryl-glycine benzyl ester as an oily substance.

### (4) Preparation of O-tert-butyl-L-seryl-O-tert-butyl-L-seryl-glycine

41g of N-benzyloxycarbonyl-O-tert-butyl-L-seryl-O-tert-butyl-L-seryl-glycine benzyl ester was added to 250g of methanol, and stirred to dissolve completely, and then 4.1g of Pd/C was added. The temperature was controlled at 23°C. The system was replaced with nitrogen, and then hydrogen was introduced. After reacting for 23 hours, TLC showed that N-benzyloxycarbonyl-O-tert-butyl-L-seryl-O-tert-butyl-L-seryl-glycine benzyl ester had completely reacted. After filtering out the Pd/C, the filtrate was concentrated until a large amount of solid precipitated. The system was cooled to 2°C for crystallization for 3 hours. The filter cake was rinsed with a small amount of methanol, and dried by blowing at 50°C to dryness to obtain 20g of O-tert-butyl-L-seryl-O-tert-butyl-L-seryl-glycine. The results show that: molar yield: 16%; HPLC: 99.28%; isomers: not detected.

### Comparative Example 1

This comparative Example uses N-benzyloxycarbonyl-O-tert-butyl-L-serine, O-tert-butyl-L-serine methyl ester and glycine methyl ester hydrochloride as main raw materials, and requires multiple synthesis steps. The obtained final compound O-tert-butyl-L-seryl-O-tert-butyl-L-seryl-glycine contains more impurities, is not easy to purify, and has a lower molar yield. This comparative Example has one more reaction step than the inventive examples.

### (1) Preparation of N-benzyloxycarbonyl-O-tert-butyl-L-seryl-O-tert-butyl-L-serine methyl ester

100g of N-benzyloxycarbonyl-O-tert-butyl-L-serine was added into 800g of ethyl acetate, and stirred to dissolve completely, and then 90g of O-tert-butyl-L-serine methyl ester and 51.3g of trimethylamine were added. After stirring uniformly, the mixture was cooled to 3°C, and then 9.1g of HOBT and 78g of EDCl were added. The mixture was stirred to react for 30 minutes, and heated to 33°C to react for 18 hours. TLC showed that N-carbobenzoxy-O-tert-butyl-L-serine had completely reacted. 400g of water was added to the reaction solution to quench the reaction, and then liquids were separated. Ethyl acetate layer was collected. The ethyl acetate layer was washed with 6N hydrochloric acid aqueous solution, 5% sodium bicarbonate aqueous solution and saturated sodium chloride aqueous solution respectively. After the washing was completed, the ethyl acetate layer was dried over sodium sulfate, filtered, and concentrated to dryness to obtain 167g of N-benzyloxycarbonyl-O-tert-butyl-L-seryl-O-tert-butyl-L-serine methyl ester as an oily substance.

### (2) Preparation of N-benzyloxycarbonyl-O-tert-butyl-L-seryl-O-tert-butyl-L-serine

167g of the N-benzyloxycarbonyl-O-tert-butyl-L-seryl-O-tert-butyl-L-serine methyl ester as the oily substance was added into 150g of tetrahydrofuran and 300g of water. The mixture was cooled to 4°C, and 67.6g of 30% sodium hydroxide aqueous solution was slowly added. After stirring uniformly, the mixture was heated to 23°C to react for 3 hours. TLC showed that N-benzyloxycarbonyl-O-tert-butyl-L-seryl-O-tert-butyl-L-serine methyl ester had completely reacted. 400g of ethyl acetate was added into the system. The pH value of the system was adjusted to 2-3 by using a 6N hydrochloric acid aqueous solution, and then liquids were separated. The ethyl acetate layer was collected. The ethyl acetate layer was washed with saturated sodium chloride for 3 times. After the washing was completed, the ethyl acetate layer was dried over sodium sulfate, filtered, and concentrated to dryness to obtain 153g of the N-benzyloxycarbonyl-O-tert-butyl-L-seryl-O-tert-butyl-L-serine as an oily substance.

### (3) Preparation of N-benzyloxycarbonyl-O-tert-butyl-L-seryl-O-tert-butyl-L-seryl-glycine methyl ester

153g of N-benzyloxycarbonyl-O-tert-butyl-L-seryl-O-tert-butyl-L-serine was added into 1200g of ethyl acetate, and stirred to dissolve completely, and then 52.5g of glycine methyl ester hydrochloride and 52.8g of triethylamine were added. After stirring uniformly, the mixture was cooled to 2°C, and then 9.4g of HOBT and 79.9g of EDCl were added. The mixture was stirred to react for 30 minutes, and heated to 33°C to react for 18 hours. TLC showed that N-benzyloxycarbonyl-O-tert-butyl-L-seryl-O-tert-butyl-L-serine had completely reacted. 400g of water was added to the reaction solution to quench the reaction, and then liquids were separated. Ethyl acetate layer was collected. The ethyl acetate layer was washed with 6N hydrochloric acid aqueous solution, 5% sodium bicarbonate aqueous solution and saturated sodium chloride aqueous solution respectively. After the washing was completed, the ethyl acetate layer was dried over sodium sulfate, filtered, and concentrated to dryness to obtain 157g of N-benzyloxycarbonyl-O-tert-butyl-L-seryl-O-tert-butyl-L-seryl- glycine methyl ester as an oily substance.

### (4) Preparation of N-benzyloxycarbonyl-O-tert-butyl-L-seryl-O-tert-butyl-L-seryl-glycine

157g of the N-benzyloxycarbonyl-O-tert-butyl-L-seryl-O-tert-butyl-L-seryl-glycine methyl ester as the oily substance was added into 160g of tetrahydrofuran and 300g of water. The mixture was cooled to 4°C, and 61.7g of 30% sodium hydroxide aqueous solution was slowly added. After stirring uniformly, the mixture was heated to 23°C to react for 3 hours. TLC showed that N-benzyloxycarbonyl-O-tert-butyl-L-seryl-O-tert-butyl-L-seryl-glycine methyl ester had completely reacted. 400g of ethyl acetate was added into the system. The pH value of the system was adjusted to 2-3 by using a 6N hydrochloric acid aqueous solution, and then liquids were separated. The ethyl acetate layer was collected. The ethyl acetate layer was washed with saturated sodium chloride for 3 times. After the washing was completed, the ethyl acetate layer was dried over sodium sulfate, filtered, and concentrated to dryness to obtain 138g of N-benzyloxycarbonyl-O-tert-butyl-L-seryl-O-tert-butyl-L-seryl- glycine as an oily substance.

### (5) Preparation of O-tert-butyl-L-seryl-O-tert-butyl-L-seryl-glycine

138g of N-benzyloxycarbonyl-O-tert-butyl-L-seryl-O-tert-butyl-L-seryl-glycine was added to 828g of methanol, and stirred to dissolve completely, and then 13.8g of Pd/C was added. The temperature was controlled at 23°C. The system was replaced with nitrogen, and then hydrogen was introduced. After reacting for 23 hours, TLC showed that N-benzyloxycarbonyl-O-tert-butyl-L-seryl-O-tert-butyl-L-seryl-glycine had completely reacted. After filtering out the Pd/C, the filtrate was concentrated until a large amount of solid precipitated. The system was cooled to 2°C for crystallization for 3 hours. The filter cake was rinsed with a small amount of methanol, and dried by blowing at 50°C to dryness to obtain 45g of O-tert-butyl-L-seryl-O-tert-butyl-L-seryl-glycine. The results show that: molar yield: 37%; HPLC: 93.6%; isomers: not detected.

### Comparative Example 2

This comparative Example uses N-tert-butoxycarbonyl-O-tert-butyl-L-serine, O-tert-butyl-L-serine methyl ester and glycine methyl ester hydrochloride as main raw materials, and requires multiple synthesis steps. The obtained final compound O-tert-butyl-L-seryl-O-tert-butyl-L-seryl-glycine contains more impurities, is not easy to purify, and has a lower molar yield. This comparative Example has one more reaction step than the inventive examples.

### (1) Preparation of N-tert-butoxycarbonyl-O-tert-butyl-L-seryl-O-tert-butyl-L-serine methyl ester

100g of N-tert-butoxycarbonyl-O-tert-butyl-L-serine was added into 800g of ethyl acetate, and stirred to dissolve completely, and then 100g of O-tert-butyl-L-serine methyl ester and 54.2g of trimethylamine were added. After stirring uniformly, the mixture was cooled to 3°C, and then 7.5g of HOBT and 88g of EDCl were added. The mixture was stirred to react for 30 minutes, and heated to 33°C to react for 18 hours. TLC showed that N-carbobenzoxy-O-tert-butyl-L-serine had completely reacted. 400g of water was added to the reaction solution to quench the reaction, and then liquids were separated. Ethyl acetate layer was collected. The ethyl acetate layer was washed with 6N hydrochloric acid aqueous solution, 5% sodium bicarbonate aqueous solution and saturated sodium chloride aqueous solution respectively. After the washing was completed, the ethyl acetate layer was dried over sodium sulfate, filtered, and concentrated to dryness to obtain 157g of N-tert-butoxycarbonyl-O-tert-butyl-L-seryl-O-tert-butyl-L-serine methyl ester as an oily substance.

### (2) Preparation of N-tert-butoxycarbonyl-O-tert-butyl-L-seryl-O-tert-butyl-L-serine

157g of the N-tert-butoxycarbonyl-O-tert-butyl-L-seryl-O-tert-butyl-L-serine methyl ester as the oily substance was added into 150g of tetrahydrofuran and 300g of water. The mixture was cooled to 4°C, and 75g of 30% sodium hydroxide aqueous solution was slowly added. After stirring uniformly, the mixture was heated to 23°C to react for 3 hours. TLC showed that N-tert-butoxycarbonyl-O-tert-butyl-L-seryl-O-tert-butyl-L-serine methyl ester had completely reacted. 400g of ethyl acetate was added into the system. The pH value of the system was adjusted to 2-3 by using a 6N hydrochloric acid aqueous solution, and then liquids were separated. The ethyl acetate layer was collected. The ethyl acetate layer was washed with saturated sodium chloride for 3 times. After the washing was completed, the ethyl acetate layer was dried over sodium sulfate, filtered, and concentrated to dryness to obtain 136g of N-tert-butoxycarbonyl-O-tert-butyl-L-seryl-O-tert-butyl-L-serine as an oily substance.

### (3) Preparation of N-tert-butoxycarbonyl-O-tert-butyl-L-seryl-O-tert-butyl-L-seryl-glycine methyl ester

136g of N-tert-butoxycarbonyl-O-tert-butyl-L-seryl-O-tert-butyl-L-serine was added into 1100g of ethyl acetate, and stirred to dissolve completely, and then 50.7g of glycine methyl ester hydrochloride and 47.6g of triethylamine were added. After stirring uniformly, the mixture was cooled to 2°C, and then 9g of HOBT and 77g of EDCl were added. The mixture was stirred to react for 30 minutes, and heated to 33°C to react for 18 hours. TLC showed that N-tert-butoxycarbonyl-O-tert-butyl-L-seryl-O-tert-butyl-L-serine had completely reacted. 400g of water was added to the reaction solution to quench the reaction, and then liquids were separated. Ethyl acetate layer was collected. The ethyl acetate layer was washed with 6N hydrochloric acid aqueous solution, 5% sodium bicarbonate aqueous solution and saturated sodium chloride aqueous solution respectively. After the washing was completed, the ethyl acetate layer was dried over sodium sulfate, filtered, and concentrated to dryness to obtain 148g of N-tert-butoxycarbonyl-O-tert-butyl-L-seryl-O-tert-butyl-L-seryl-glycine methyl ester as an oily substance.

### (4) Preparation of N-tert-butoxycarbonyl-O-tert-butyl-L-seryl-O-tert-butyl-L-seryl-glycine

148g of the N-tert-butoxycarbonyl-O-tert-butyl-L-seryl-O-tert-butyl-L-seryl-glycine methyl ester as the oily substance was added into 150g of tetrahydrofuran and 300g of water. The mixture was cooled to 4°C, and 62.3g of 30% sodium hydroxide aqueous solution was slowly added. After stirring uniformly, the mixture was heated to 23°C to react for 3 hours. TLC showed that N-tert-butoxycarbonyl-O-tert-butyl-L-seryl-O-tert-butyl-L-seryl-glycine methyl ester had completely reacted. 400g of ethyl acetate was added into the system. The pH value of the system was adjusted to 2-3 by using a 6N hydrochloric acid aqueous solution, and then liquids were separated. The ethyl acetate layer was collected. The ethyl acetate layer was washed with saturated sodium chloride for 3 times. After the washing was completed, the ethyl acetate layer was dried over sodium sulfate, filtered, and concentrated to dryness to obtain 132g of N-tert-butoxycarbonyl-O-tert-butyl-L-seryl-O-tert-butyl-L-seryl-glycine as an oily substance.

### (5) Preparation of O-tert-butyl-L-seryl-O-tert-butyl-L-seryl-glycine

132g of N-tert-butoxycarbonyl-O-tert-butyl-L-seryl-O-tert-butyl-L-seryl-glycine was added to 660g of dichloromethane, and stirred to dissolve completely, and then 65.3g of trifluoroacetic acid was added. The temperature was controlled at 23°C. After reacting for 4 hours, TLC showed that N-tert-butoxycarbonyl-O-tert-butyl-L-seryl-O-tert-butyl-L-seryl-glycine had completely reacted. The dichloromethane and trifluoroacetic acid were concentrated to dryness. Then 500g of methanol was added. The system was cooled to 7°C. The pH value of the system was adjusted to 6-7 by using 35g of triethylamine. Then the system was cooled to 2°C for crystallization for 3 hours, and then filtered. The filter cake was rinsed with a small amount of methanol, and dried by blowing at 50°C to dryness to obtain 34g of O-tert-butyl-L-seryl-O-tert-butyl-L-seryl-glycine. The results show that: molar yield: 28%; HPLC: 95.3%; isomers: not detected.

Table 1 provides data on the yield and purity under different raw material selections for Example 1, Comparative Example 1, and Comparative Example 2.

**Table 1 Data on Effect of Different Main Raw Materials on the Yield and Purity of the Target Product**

| | Main Raw Material 1 | Main Raw Material 2 | Main Raw Material 3 | Yiel d | Purity |
|---|---|---|---|---|---|
| Example 1 | N-benzyloxycarbon yl-O-tert-butyl-L-serine | O-tert-butyl-L-seri ne methyl ester | Glycine benzyl ester p-toluenesulfonate | 73% | 99.48 % |
| Comparative Example 1 | N-benzyloxycarbon yl-O-tert-butyl-L-s erine | O-tert-butyl-L-seri ne methyl ester | Glycine methyl ester hydrochloride | 37% | 93.6% |
| Comparative Example 2 | N-tert-butoxycarbo nyl-O-tert-butyl-L-serine | O-tert-butyl-L-seri ne methyl ester | Glycine methyl ester hydrochloride | 28% | 95.3% |

### Example 5

Example 5 investigates the effect of condensing agents on the target product. Specifically, the preparation method is the same as that of Example 1, except that the types of the condensing agents in step (1) and step (3) are changed. Through experiments, the yield and purity of the obtained target product are shown in Table 2 below.

**Table 2 Data on Yield and Purity of the Target Product Under Different Condensing Agent Selections**

| | Condensing Agent | Yield | Purity |
|---|---|---|---|
| Experimental Group 1 (Example 1) | EDCl/HOBT | 73% | 99.48% |
| Experimental Group 2 | EDCl/HOSU | 68% | 99.37% |
| Experimental Group 3 | DCC/HOSU | 54% | 97.68% |
| Experimental Group 4 | HATU | 69% | 99.33% |
| Experimental Group 5 | HBTU | 68% | 99.36% |
| Experimental Group 6 | CDI | 38% | 98.42% |

### Example 6

Example 6 investigates the effect of temperature of condensation reaction on the target product. Specifically, the preparation method is the same as that of Example 1, except that the temperature of the condensation reaction is changed. Through experiments, the yield and purity of the obtained target product are shown in Table 3 below.

**Table 3 Data on Yield and Purity of the Target Product at Different Condensation Temperatures**

| | Temperature of Condensation Reaction /°C | Yield | Purity |
|---|---|---|---|
| Experimental Group 7 (Example 4) | 0-10 | 16% | 99.28% |
| Experimental Group 8 | 10-20 | 32% | 99.34% |
| Experimental Group 9 | 20-25 | 54% | 99.44% |
| Experimental Group 10 | 25-30 | 64% | 99.41% |
| Experimental Group 1 (Example 1) | 30-35 | 73% | 99.48% |
| Experimental Group 11 | 35-40 | 75% | 98.42% |
| Experimental Group 12 | 40-50 | 74% | 96.26% |

### Example 7

Example 7 investigates the effect of organic solvents on the target product. Specifically, the preparation method is the same as that of Example 1, except that the selection of organic solvents in step (1) and step (3) is changed. Through experiments, the yield and purity of the obtained target product are shown in Table 4 below.

**Table 4 Data on Yield and Purity of the Target Product with Different Organic Solvents**

| | Organic Solvent | Yield | Purity |
|---|---|---|---|
| Experimental Group 1 (Example 1) | Ethyl Acetate | 73% | 99.48% |
| Experimental Group 13 | Tetrahydrofuran | 55% | 99.14% |
| Experimental Group 14 | Dichloromethane | 46% | 98.44% |
| Experimental Group 15 | Acetonitrile | 62% | 99.06% |

### Example 8

Example 8 investigates the effect of concentration of sodium hydroxide on the target product. Specifically, the preparation method is the same as that of Example 1, except that the concentration of sodium hydroxide solution in step (2) is changed. Through experiments, the reaction time (the reaction time of step (2)) and the purity of the obtained target product are shown in Table 5 below.

**Table 5 Data on Reaction Time and Product Purity at Different Concentrations of Sodium Hydroxide Solution**

| | Concentration of Sodium Hydroxide | Reaction Time | Purity |
|---|---|---|---|
| Experimental Group 16 | 10% Sodium Hydroxide | 7h | 99.38% |
| Experimental Group 17 | 20% Sodium Hydroxide | 5h | 99.42% |
| Experimental Group 1 (Example 1) | 30% Sodium Hydroxide | 3h | 99.48% |
| Experimental Group 18 | 40% Sodium Hydroxide | 2h | 98.11% |
| Experimental Group 19 | 50% Sodium Hydroxide | 1h | 95.54% |
| Experimental Group 20 | 60% Sodium Hydroxide | 0.5h | 92.16% |

### Example 9

Example 9 investigates the effect of temperature of saponification reaction on the target product. Specifically, the preparation method is the same as that of Example 1, except that the temperature of the saponification reaction in step (2) is changed. Through experiments, the reaction time (the reaction time of step (2)) and the purity of the obtained target product are shown in Table 6 below.

**Table 6 Data on Reaction Time and Product Purity at Different Temperatures of the Saponification Reaction**

| | Temperature of Saponification Reaction/°C | Reaction Time | Purity |
|---|---|---|---|
| Experimental Group 21 | 0-10 | 6h | 99.48% |
| Experimental Group 22 | 10-15 | 5h | 99.46% |
| Experimental Group 23 | 15-20 | 4h | 99.49% |
| Experimental Group 1 (Example 1) | 20-25 | 3h | 99.48% |
| Experimental Group 24 | 25-30 | 2h | 98.37% |
| Experimental Group 25 | 30-40 | 1h | 96.74% |

In summary, the present application provides a method for preparing the tirzepatide intermediate O-tert-butyl-L-seryl-O-tert-butyl-L-seryl-glycine, which is suitable for scaled-up production. The synthesis method requires a simple equipment, has a good reaction efficiency, and a low production cost, and the final product has a purity of higher than 99.0%.

The above description of the examples is only used to help understand the method of the present application and its core idea. It should be pointed out that for those of ordinary skill in the technical field, several improvements and modifications can be made to the present application without departing from the principles of the present application, and these improvements and modifications also fall within the protection scope of the claims of the present application.

The above description of the examples enables those skilled in the art to implement or use the present application. Various modifications to these examples will be obvious to those skilled in the art, and the general principles defined herein can be implemented in other examples without departing from the spirit or scope of the present application. Therefore, the present application will not be limited to the examples shown herein, but is to conform to the widest scope consistent with the principles and novel features disclosed herein.

## Claims

1. O-tert-butyl-L-seryl-O-tert-butyl-L-seryl-glycine represented by formula (I)

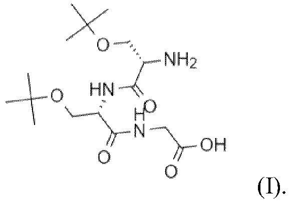

2. A method for preparing O-tert-butyl-L-seryl-O-tert-butyl-L-seryl-glycine, comprising the following steps:
S1) carrying out a condensation reaction on N-benzyloxycarbonyl-O-tert-butyl-L-serine and O-tert-butyl-L-serine methyl ester under the action of a condensing agent and an organic base to obtain N-benzyloxycarbonyl-O-tert-butyl-L-seryl-O-tert-butyl-L-serine methyl ester represented by formula (II);
S2) carrying out a saponification reaction on the N-benzyloxycarbonyl-O-tert-butyl-L-seryl-O-tert-butyl-L-serine methyl ester and an alkaline compound to obtain N-benzyloxycarbonyl-O-tert-butyl-L-seryl-O-tert-butyl-L-serine represented by formula (III);
S3) carrying out a condensation reaction on the N-benzyloxycarbonyl-O-tert-butyl-L-seryl-O-tert-butyl-L-serine and glycine benzyl ester p-toluenesulfonate under the action of a condensing agent and an organic base to obtain N-benzyloxycarbonyl-O-tert-butyl-L-seryl-O-tert-butyl-L-seryl-glycine benzyl ester represented by formula (IV);
S4) carrying out a hydrogenation reaction on the N-benzyloxycarbonyl-O-tert-butyl-L-seryl-O-tert-butyl-L-seryl-glycine benzyl ester and hydrogen under the action of a catalyst to obtain O-tert-butyl-L-seryl-O-tert-butyl-L-seryl-glycine represented by formula (I);

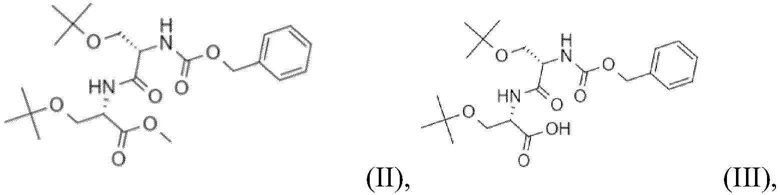

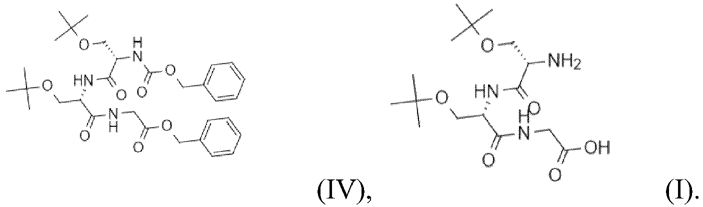

3. The method according to claim 2, wherein step S1) specifically is:
mixing the N-benzyloxycarbonyl-O-tert-butyl-L-serine, the O-tert-butyl-L-serine methyl ester and the organic base with an organic solvent, then adding the condensing agent to carry out the condensation reaction to obtain the N-benzyloxycarbonyl-O-tert-butyl-L-seryl-O-tert-butyl-L-serine methyl ester.

4. The method according to claim 2 or 3, wherein in step S1), a molar ratio of the N-benzyloxycarbonyl-O-tert-butyl-L-serine, the O-tert-butyl-L-serine methyl ester, the condensing agent, and the organic base is 1: (1-2): (1-1.5): (1-1.5).

5. The method according to claim 2 or 3, wherein the condensing agent is selected from the group consisting of EDCl/HOBT, EDCl/HOSU, DCC/HOSU, HATU, HBTU and CDI, and the organic base is selected from the group consisting of triethylamine and N, N-diisopropylethylamine.

6. The method according to claim 3, wherein the condensing agent is added at a temperature of 0-5°C, and the condensation reaction is carried out at a temperature of 0-50°C for 12-24 h; or
wherein the organic solvent is selected from the group consisting of ethyl acetate, dichloromethane, acetonitrile and tetrahydrofuran.

7. The method according to claim 2, wherein step S2) specifically is:
mixing the N-benzyloxycarbonyl-O-tert-butyl-L-seryl-O-tert-butyl-L-serine methyl ester with an organic solvent, then adding a sodium hydroxide solution to carry out the saponification reaction to obtain the N-benzyloxycarbonyl-O-tert-butyl-L-seryl-O-tert-butyl-L-serine.

8. The method according to claim 7, wherein in step S2), a molar ratio of the N-benzyloxycarbonyl-O-tert-butyl-L-seryl-O-tert-butyl-L-serine methyl ester to the sodium hydroxide is 1: (1-2); or
wherein in step S2), the sodium hydroxide solution is added at a temperature of 0-5°C, and the saponification reaction is carried out at a temperature of 0-40°C for 1-6 h; or
wherein in step S2), a concentration of the sodium hydroxide solution is 5-60%; or
wherein in step S2), the organic solvent is selected from the group consisting of acetonitrile, tetrahydrofuran, methanol and ethanol.

9. The method according to claim 2, wherein step S3) specifically is:
mixing the N-benzyloxycarbonyl-O-tert-butyl-L-seryl-O-tert-butyl-L-serine, the glycine benzyl ester p-toluenesulfonate and the organic base with an organic solvent, then adding the condensing agent to carry out the condensation reaction to obtain the N-benzyloxycarbonyl-O-tert-butyl-L-seryl-O-tert-butyl-L-seryl-glycine benzyl ester.

10. The method according to claim 2 or 9, wherein in step S3), a molar ratio of the N-benzyloxycarbonyl-O-tert-butyl-L-seryl-O-tert-butyl-L-serine, the glycine benzyl ester p-toluenesulfonate, the organic base, and the condensing agent is 1: (1-2): (1-1.5): (1-1.5).

11. The method according to claim 2 or 9, wherein in step S3), the condensing agent is selected from the group consisting of EDCl/HOBT, EDCl/HOSU, DCC/HOSU, HATU, HBTU and CDI, and the organic base is selected from the group consisting of trimethylamine and N, N-diisopropylethylamine.

12. The method according to claim 9, wherein in step S3), the condensing agent is added at a temperature of 0-5°C, and the condensation reaction is carried out at a temperature of 0-50°C for 12-24 h; or
wherein in step S3), the organic solvent is selected from the group consisting of ethyl acetate, dichloromethane, acetonitrile and tetrahydrofuran.

13. The method according to claim 2, wherein step S4) specifically is:
mixing the N-benzyloxycarbonyl-O-tert-butyl-L-seryl-O-tert-butyl-L-seryl-glycine benzyl ester with an organic solvent, adding the catalyst, and then introducing hydrogen to carry out the hydrogenation reaction to obtain the O-tert-butyl-L-seryl-O-tert-butyl-L-seryl-glycine.

14. The method according to claim 2 or 13, wherein in step S4), a mass ratio of the N-benzyloxycarbonyl-O-tert-butyl-L-seryl-O-tert-butyl-L-seryl-glycine benzyl ester to the catalyst is 1: (0.01-0.2); or
wherein in step S4), the hydrogenation reaction is carried out at a temperature of 20-30°C for 20-24 h.

15. The method according to claim 13, wherein the organic solvent is selected from the group consisting of acetonitrile, tetrahydrofuran/water, methanol, ethanol, acetone/water and ethyl acetate/water, and the catalyst is selected from the group consisting of Pd/C, Raney nickel and Pd/BaSO₄.
